(19) **Europäisches Patentamt / European Patent Office / Office européen des brevets**

(11) **EP 2 412 379 A1**

(12) **EUROPEAN PATENT APPLICATION**
published in accordance with Art. 153(4) EPC

(43) Date of publication:
**01.02.2012 Bulletin 2012/05**

(21) Application number: **10756235.7**

(22) Date of filing: **26.03.2010**

(51) Int Cl.:
*A61K 38/00* (2006.01)    *A61P 1/14* (2006.01)
*A61P 25/02* (2006.01)    *A61P 25/22* (2006.01)
*A61P 25/28* (2006.01)

(86) International application number:
**PCT/JP2010/055413**

(87) International publication number:
**WO 2010/110439 (30.09.2010 Gazette 2010/39)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **27.03.2009 JP 2009078015**

(71) Applicant: **Calpis Co., Ltd.**
**Tokyo 150-0022 (JP)**

(72) Inventors:
• **UCHIDA Naoto**
**Sagamihara-shi**
**Kanagawa 252-0206 (JP)**
• **OHSAWA Kazuhito**
**Sagamihara-shi**
**Kanagawa 252-0206 (JP)**
• **OHKI Kohji**
**Sagamihara-shi**
**Kanagawa 252-0206 (JP)**
• **NAKAMURA Yasunori**
**Sagamihara-shi**
**Kanagawa 252-0206 (JP)**
• **BABA Hidehiko**
**Sagamihara-shi**
**Kanagawa 252-0206 (JP)**

(74) Representative: **Denison, Christopher Marcus et al**
**Mewburn Ellis LLP**
**33 Gutter Lane**
**GB-London EC2V 8AS (GB)**

(54) **COMPOSITION FOR REGULATING AUTONOMIC NERVOUS ACTIVITY AND METHOD FOR REGULATING AUTONOMIC NERVE**

(57) The present invention provides an orally ingestible composition and a method for regulating an autonomic nervous activity. In particular, the present invention provides an orally ingestible composition capable of suppressing an increase in a sympathetic nervous activity, and an orally ingestible composition capable of promoting a parasympathetic nervous activity. The present invention is a composition for regulating an autonomic nervous activity, comprising, as an active ingredient, Tyr-Pro or a salt thereof.

EP 2 412 379 A1

**Description**

**FIELD OF THE INVENTION**

The present invention relates to a composition for regulating an autonomic nervous activity and a method for regulating an autonomic nervous activity. In particular, the present invention relates to a composition for suppressing an increase in a sympathetic nervous activity, and a composition for promoting a parasympathetic nervous activity. Moreover, the present invention relates to a method for suppressing an increase in a sympathetic nervous activity, and a method for promoting a parasympathetic nervous activity.

**BACKGROUND OF THE INVENTION**

The autonomic nervous system is a nervous system which constitutes a part of nervous systems which are not regulated consciously and controls the cardiac muscle, the smooth muscle and the gland tissues, in contrast to the somatic motor nervous system, which mainly controls the skeletal muscle. The autonomic nervous system is different from the motor nervous system also in terms of structure and is constituted of two kinds of nerve cells called the preganglionic nerve and the postganglionic nerve. The autonomic nervous system is broadly classified into the sympathetic nervous system and the parasympathetic nervous system, which have mutually different functions. It has been known that the activity of the sympathetic nerve generally causes an increase in heart rate, an elevation in blood pressure, dilatation of the pupillary, liberation of glucose into blood, and the like, whereas the activity of the parasympathetic nerve generally increases the gastrointestinal motility, stimulates the secretion of digestive fluids and the like, and reduces the heart rate. The two nervous systems cooperate to play important roles in maintaining the homeostasis of living organisms.
As a report on the direct measurement of an influence of a peptide or a peptide derivative on the autonomic nervous activity, an effect of controlling the autonomic nerve of carnosine has been reported (Document 1). However, since this effect is evaluated through intravenous administration, the effect through oral administration has been unknown. Moreover, there has been no report on the direct measurement of an influence of the dipeptide Tyr-Pro (YP) or an analog thereof on an autonomic nervous activity. Meanwhile, there have been some reports on the physiological functions of Tyr-Pro and analogs thereof. For example, it is known through a test using extirpated ileal longitudinal muscle that some kinds of penta peptides having Tyr-Pro- in the N-terminus portion have an opioid activity to alleviate pain, and are effective in controlling emotions, and in regulating respiration, body temperature and digestive activities (Document 2). Moreover, Tyr-Pro is known to exhibit an analgesic effect when administrated intraperitoneally to a rat, although Tyr-Pro lacks binding capability to opioid receptors (Document 3). Moreover, it has been reported that some peptides having 4 to 9 residues including Tyr-Pro regulate an effect of a stress-induced neurotransmitter (Document 4). However, none of these reports shows any examples of direct measurement of an influence of a peptide on the autonomic nervous activity, and provides no specific report focusing on the regulatory effect thereof. Moreover, these reports relate to the effects on extirpated digestive organs or effects on living organism through intraperitoneal injection, and hence fail to demonstrate orally ingestible substances producing sufficient effects. Meanwhile, orally administered Tyr-Pro is also known to have an effect of lowering the blood pressure (Document 5). However, effects related to the regulation of autonomic nerve activities have also been unknown.

**SUMMARY OF THE INVENTION**

The present invention provides an orally ingestible composition for regulating an autonomic nervous activity. Moreover, the present invention provides a method for regulating an autonomic nervous activity.
Particularly, the present invention provides an orally ingestible composition for suppressing an increase in a sympathetic nervous activity, and an orally ingestible composition for promoting a parasympathetic nervous activity.

(1) The present invention is a composition for regulating an autonomic nervous activity, comprising, as an active ingredient, Tyr-Pro or a salt thereof.
(2) The present invention is also a composition for promoting a parasympathetic nervous activity, comprising, as an active ingredient, Tyr-Pro or a salt thereof.
(3) The present invention is a composition for suppressing an increase in a sympathetic nervous activity, comprising, as an active ingredient, Tyr-Pro or a salt thereof.
(4) Further, the present invention is the composition according to (2) or (3), wherein the composition has an effect of promoting gastrointestinal motility, an effect of preventing amnesia, or an anti-anxiety effect.
(5) The present invention is also a composition for promoting gastrointestinal motility, comprising, as an active ingredient, Tyr-Pro or a salt thereof.
(6) The present invention is also a composition for preventing amnesia comprising, as an active ingredient, Tyr-Pro or a salt thereof.

(7) The present invention is also a composition for reducing anxiety, comprising, as an active ingredient, Tyr-Pro or a salt thereof.

(8) The present invention is also the composition according to any one of (1) to (7), wherein the composition is for oral ingestion.

(9) Further, the present invention is a method for regulating an autonomic nervous activity in a non-human animal, comprising administering to a non-human animal Tyr-Pro or a salt thereof.

(10) The present invention is also a method for promoting a parasympathetic nervous activity in a non-human animal, comprising administering to a non-human animal Tyr-Pro or a salt thereof.

(11) The present invention is also a method for suppressing an increase in a sympathetic nervous activity in a non-human animal, comprising administering to a non-human animal Tyr-Pro or a salt thereof.

(12) The present invention is also the method according to (10) or (11), wherein the method is for promoting gastrointestinal motility, preventing amnesia, or reducing anxiety, in a non-human animal.

(13) The present invention is also a method for promoting gastrointestinal motility, comprising administering to a non-human animal Tyr-Pro or a salt thereof.

(14) The present invention is also a method for preventing amnesia, comprising administering to a non-human animal Tyr-Pro or a salt thereof.

(15) The present invention is also a method for reducing anxiety, comprising administering to a non-human animal Tyr-Pro or a salt thereof.

(16) The present invention is also the method according to any one of claims 9 to 15, wherein the administering is oral administration.

**BRIEF DESCRIPTION OF THE DRAWINGS**

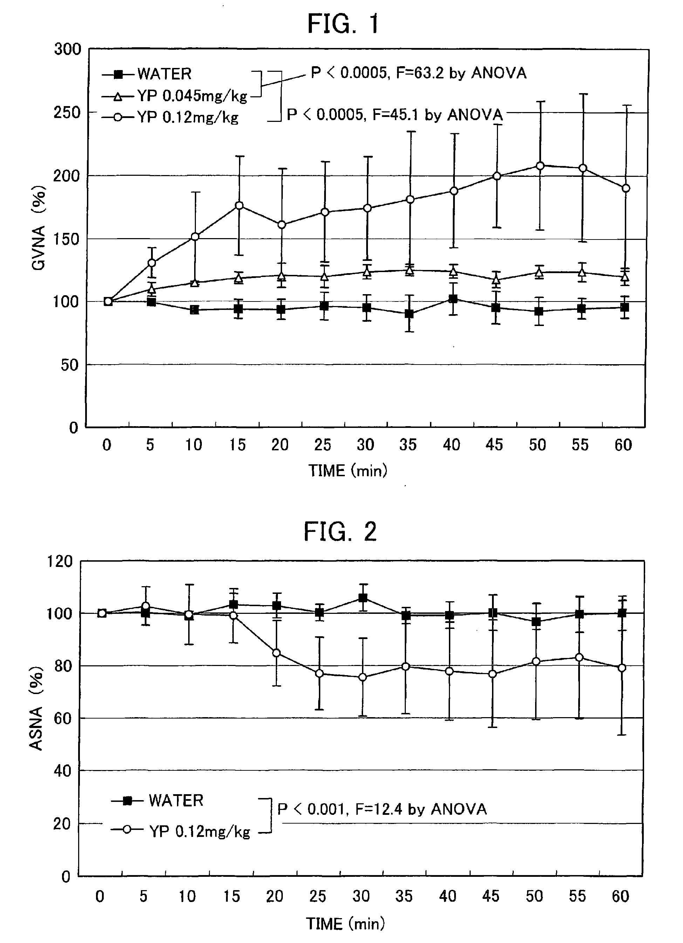

Fig. 1 shows an effect of the peptide Tyr-Pro (YP) on a parasympathetic nervous activity in the stomach. Water or 0.045 mg/kg weight or 0.12 mg/kg weight of Tyr-Pro was administered to rats (n=3), and the action potential of the parasympathetic (vagus) nerve controlling the stomach was measured over time. The vertical axis (GVNA: Gastric Vagus Nerve Activity) shows the percentage of relative action potential for each experimental group, where the value before the administration (0-minute value) is taken as 100. The horizontal axis shows the time (minutes) elapsed after the sample administration. Values for 5 to 60 minutes after the administration were treated as a group, and a significance test with respect to the water-administered control group was conducted by the analysis of variance (ANOVA).

Fig. 2 shows an effect of the peptide Tyr-Pro (YP) on a sympathetic nervous activity in the adrenal gland. Water or 0.12 mg/kg weight of Tyr-Pro was administered to rats (n=3), and the action potential of the sympathetic nerve controlling the adrenal gland was measured over time. The vertical axis (ASNA: Adrenal Sympathetic Nerve Activity) shows the percentage of relative action potential for each experimental group, where the value before the administration (0-minute value) is taken as 100. The horizontal axis shows the time (minutes) elapsed after the sample administration. Values for 5 to 60 minutes after the administration were treated as a group, and a significance test with respect to the water-administered control group was conducted by the analysis of variance (ANOVA).

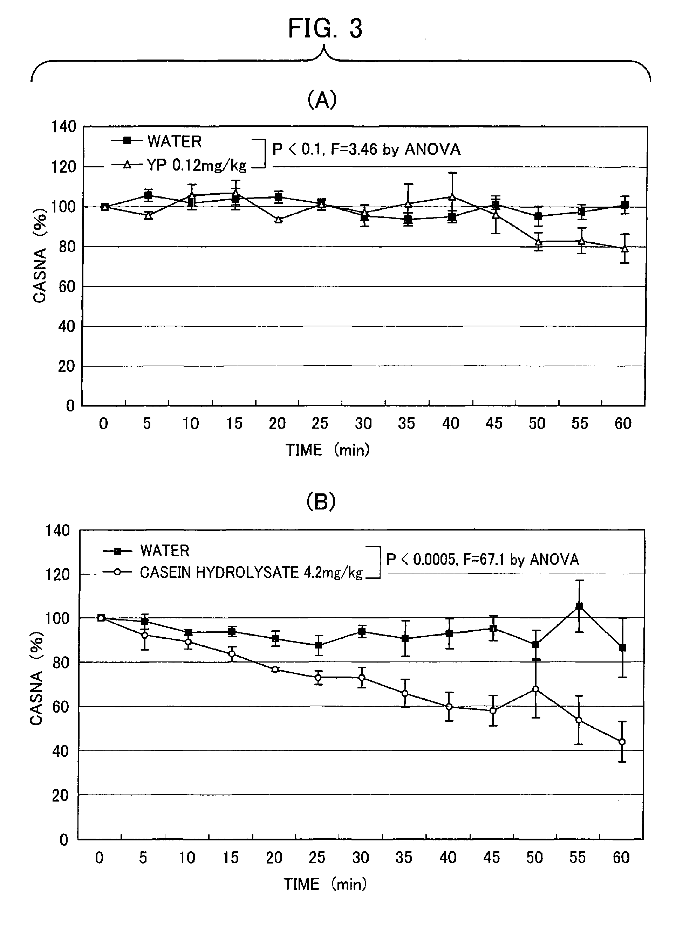

Fig. 3 shows effects of Tyr-Pro (YP) (Fig. 3A) and a casein hydrolysate (Fig. 3B) on a sympathetic nervous activity in the cutaneous artery. Water, 0.12 mg/kg weight of Tyr-Pro, or 4.2 mg/kg weight of a casein hydrolysate was administered to rats (n=3), and the action potential of the sympathetic nerve in the cutaneous artery of the tail was measured over time. The vertical axis (CASNA: Cutaneous Arterial Sympathetic Nerve Activity) shows the percentage of relative action potential for each experimental group, where the value before the administration (0-minute value) is taken as 100. The horizontal axis shows the time (minutes) after the sample administration. Values for 5 to 60 minutes after the administration were treated as a group, and a significance test with respect to the water-administered control group was conducted by the analysis of variance (ANOVA).

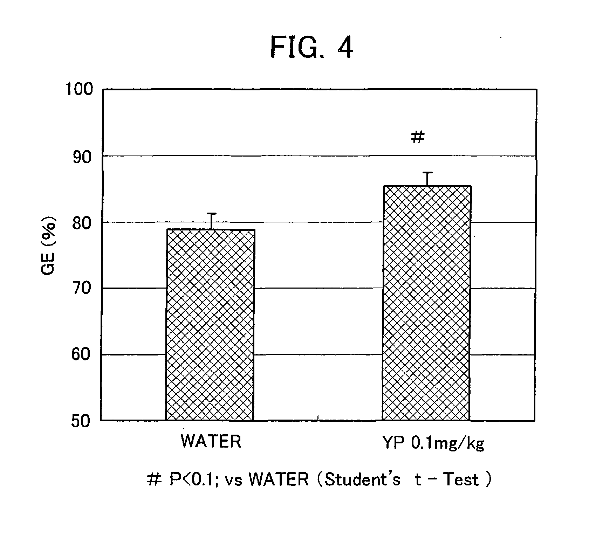

Fig. 4 shows an effect of the peptide Tyr-Pro (YP) on a gastric emptying function. Water or 0.1 mg/kg weight of Tyr-Pro was administered to rats (n=10), and 30 minutes later a dye (phenol red) was administered. After additional 20 minutes, each stomach was extirpated, and the dye remaining in the stomach was determined. A significance test with respect to the water-administered control group was conducted by an unpaired Student's t test.

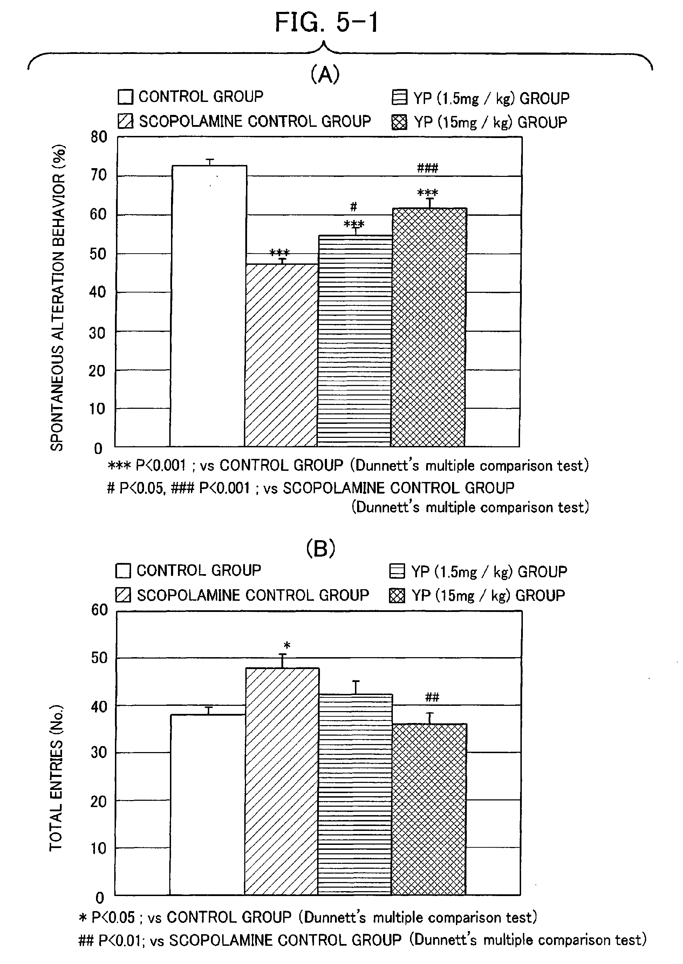

Fig. 5-1 shows an effect of preventing scopolamine-induced amnesia of the peptide Tyr-Pro (YP). Water, scopolamine alone, or 1.5 mg/kg weight or 15 mg/kg weight of Tyr-Pro together with scopolamine was administered to mice, and their respective effects of preventing amnesia were evaluated in accordance with a method described in Example 3. The vertical axis in Fig. 5-1A shows the percentage of change in spontaneous alternation behavior. The vertical axis in Fig. 5-1 B shows the total number of entries into arms. A significance test on the groups was conducted by a Dunnett's multiple comparison test. In Fig. 5-1A, *** indicates that P<0.001 with respect to the water-administered control group, # indicates that P<0.05 with respect to the group to which scopolamine alone was administered, and ### indicates that P<0.001. Meanwhile, in Fig. 5-1 B, * indicates that P<0.05 with respect to the water-administered control group, and ## indicates that P<0.01 with respect to the group to which scopolamine alone was administered.

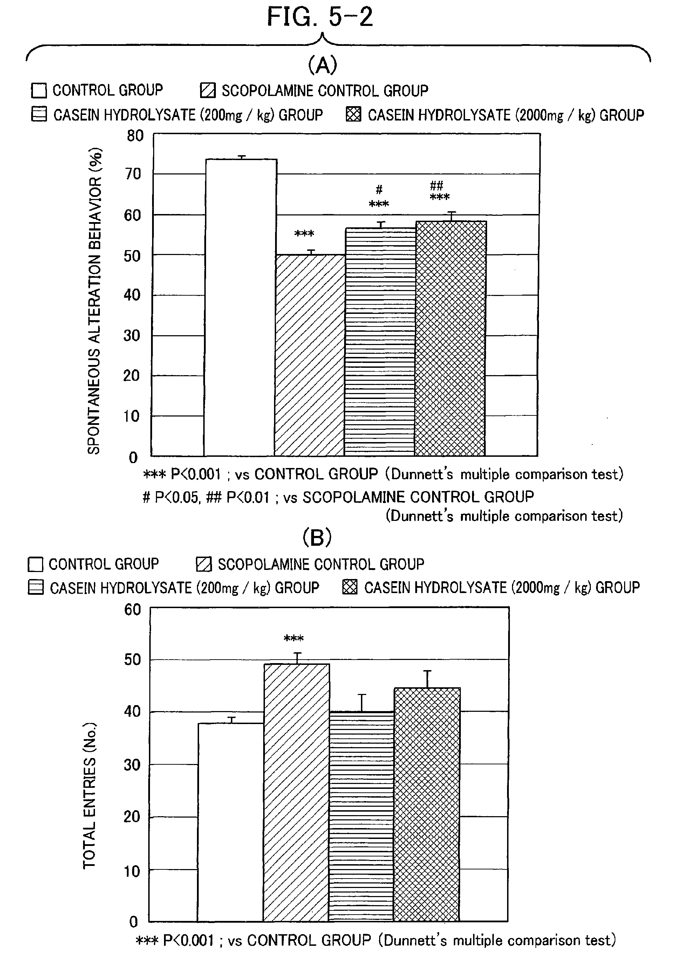

Fig. 5-2 shows an effect of preventing scopolamine-induced amnesia of a casein hydrolysate. Water, scopolamine alone, or 200 mg/kg weight or 2000 mg/kg weight of a casein hydrolysate together with scopolamine was administered to mice, and their respective effects of preventing amnesia were evaluated in accordance with a method described in Example 3. The vertical axis in Fig. 5-2A shows the percentage of change in spontaneous alternation behavior. The vertical axis in Fig. 5-2B shows the total number of entries into arms. A significance test on the groups was conducted by a Dunnett's multiple comparison test. In Fig. 5-2A, *** indicates that P<0.001 with respect to the water-administered control group, # indicates that P<0.05 with respect to the group to which scopolamine alone was administered, and ## indicates that P<0.001. Meanwhile, in Fig. 5-2B, *** indicates that P<0.001 with respect to the water-administered control group, and # indicates that P<0.05 with respect to the group to which scopolamine alone was administered.

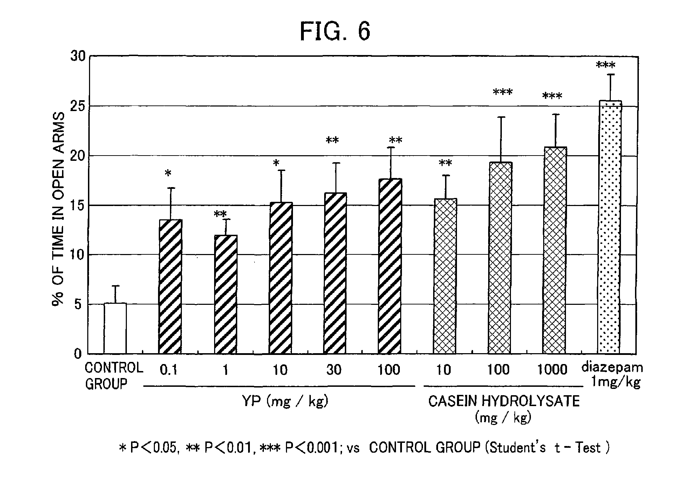

Fig. 6 shows anti-anxiety effects of the peptide Tyr-Pro (YP) and a casein hydrolysate. Water, 0.1 mg/kg weight to 100 mg/kg weight of Tyr-Pro, or 10 mg/kg to 1000 mg/kg weight of a casein hydrolysate was administered to mice (n=12), and the time for which the mice stayed on open arms was measured (Example 4). The vertical axis shows the percentage of the open arm stay time of each of the control group to which water alone was administered, the Tyr-Pro-administered groups, and the casein hydrolysate-administered groups. The data were expressed in the form of mean ± standard error. A significance test with respect to the water-administered control group was conducted by an unpaired Student's t test, and *, **, and *** indicate that P<0.05, P<0.01, and P<0.001, respectively.

## MODES FOR CARRYING OUT THE INVENTION

The peptide Tyr-Pro, which is an active ingredient of a composition of the present invention, may be an chemically-synthesized peptide or a peptide derived from a natural product. For the organic-chemical synthesis of these peptides, a commonly-used method, such as a solid phase synthesis (Boc-chemistry or Fmoc-chemistry) and a liquid phase synthesis, may be employed. For example, these peptides may be synthesized using an automated peptide synthesizer such as a peptide synthesizer (PSSM-8) available from Shimadzu Corporation. A method for the peptide synthesis, appropriate reaction conditions, and the like may be selected based on the common general technical knowledge of a person skilled in the art at the discretion of the person. A method for purifying a chemically-synthesized peptide is also well known to those skilled in the art.

As used in the specification, when referring to the peptide Tyr-Pro, "Tyr-Pro" and "the peptide Tyr-Pro" include salts thereof unless otherwise clearly indicated or otherwise obvious within the context that they should be excluded. Examples of such salts include sodium salts, potassium salts, which may exist under physiological conditions. The composition of the present invention may include other peptide and a free amino acid or a salt thereof, in addition to the peptide Tyr-Pro, which is the active ingredient of the composition of the present invention. In relation to the present invention, three-letter and single-letter codes for amino acids, and peptide notation follow the general rules well known to those skilled in the art.

In a mode of the present invention, the peptide Tyr-Pro may be derived from a fermentation product obtained by fermenting a raw material containing a protein having a Tyr-Pro sequence (for example, cow's milk, horse's milk, goat's milk, sheep's milk, skim milk powders thereof, soybean, wheat, or the like) with bacteria of the species Lactobacillus helveticus or the like. In the fermentation, the amount of the protein raw material is not particularly limited, and preferably 1 to 19% by weight, in general. Such a fermentation product may be used as a composition of the present invention directly or after appropriate treatments such as centrifugation, filtration, chromatography, drying, and the like.

In another mode of the present invention, the Tyr-Pro may be derived from a hydrolysate of a protein containing Tyr-Pro. Examples of such proteins include animal milk caseins such as caseins of cow's milk, horse's milk, sheep's milk, goat's milk, and the like, and concentrates thereof. Moreover, the Tyr-Pro may be derived from a fermentation product obtained by fermenting a raw material food containing animal milk casein with mold or bacteria such as Aspergillus or lactic acid bacteria. The concentration of the casein for the hydrolysis or fermentation of the animal milk casein is not particularly limited, and preferably 1 to 19 % by weight, in general. When a commercially available enzyme mixture is used, optimum conditions are established in general, but conditions such as the amount of enzyme used, reaction time, and the like may be changed as appropriate depending on the enzyme mixture so that the casein hydrolysate can be obtained. For example, a protein-degrading enzyme may be added to an aqueous solution in which an animal milk casein is dissolved at an enzyme mixture/animal milk casein weight ratio of 1/1000 or higher, preferably 1/100 to 1/10, particularly preferably 1/40 to 1/10. The reaction conditions may be selected as appropriate depending on the enzyme mixture used, and are generally 25 to 60°C, preferably 45 to 55°C, and a pH of 3 to 5, preferably 5 to 9. Meanwhile, the reaction time is 2 to 48 hours, and preferably 7 to 15 hours. Such a protein hydrolysate, particularly, a casein hydrolysate may be used as the composition of the present invention directly or after treatments such as centrifugation, filtration, chromatography, drying, and the like.

The composition of the present invention has an effect of regulating an autonomic nervous activity. Activities of the autonomic nervous include sympathetic nervous activities and parasympathetic nervous activities. The composition of the present invention has an effect of suppressing an increase in a sympathetic nervous activity and an effect of promoting a parasympathetic nervous activity. In particular, since the composition of the present invention suppresses an increase in a sympathetic nervous activity, or promotes a parasympathetic nervous activity, the composition consequently has an effect of promoting the gastrointestinal motility, an effect of preventing amnesia, an anti-anxiety effect, or an effect of promoting blood flow. Moreover, since the composition of the present invention has an effect of regulating the autonomic nerve, the composition has a wide range of effects such as an effect of regulating heart rate, an effect of regulating blood glucose, an effect of regulating gastric juice secretion, an effect of regulating body temperature, an effect of regulating blood pressure, an effect of stress relaxation, and the like.

The effects of regulating autonomic nervous activities, particularly, the effect of suppressing an increase in a sympathetic nervous activity, and the effect of promoting a parasympathetic nervous activity may be confirmed by the composition of the present invention and the peptide Tyr-Pro, which is the active ingredient thereof, for example, as follows.

The effect of promoting a parasympathetic nervous activity of the composition of the present invention may be confirmed, for example, on the basis of an increase in action potential observed when the present composition is administered to a rat or a mouse and the action potential of the parasympathetic (vagus) nerve controlling the stomach is measured over time. Alternatively, the effect of promoting the parasympathetic nervous activity of the present composition may be confirmed by using an enhancement of the gastric emptying function, i.e., the activation of the stomach as an index. Specifically, the composition of the present invention is administered to a rat or a mouse, and, after a certain period of time (for example, 15 minutes to 30 minutes later), a dye, for example, phenol red is introduced into the stomach of the rat or the mouse. After a certain period of time has elapsed (for example, 20 minutes later), the stomach of the rat or the mice is extirpated and the amount of the dye remaining therein is determined to confirm the effect. Meanwhile, the effect of suppressing an increase in a sympathetic nervous activity of the composition of the present invention may be confirmed on the basis of decrease in action potential observed when the present composition is administered to a rat or a mouse, and the action potential of a sympathetic nerve controlling the adrenal gland is measured over time. Moreover, the anti-anxiety effect of the present composition may be confirmed based on, for example, the elevated plus-maze test conducted on a mouse. Specifically, water, the present composition, or the peptide Tyr-Pro is administered to a mouse, and the mouse is placed on a platform at the center of an elevated maze. Then, the periods of time for which the mouse stays on open arms (wall-less arms) and on closed arms (walled arms) are measured for a certain period of time. If the period of time for which the mouse stays on the open arm is longer than that of the control group to which only water is administered, it is determined that an anti-anxiety effect is exhibited. The effect of preventing amnesia of the composition or the peptide Tyr-Pro may be confirmed using a system based on an evaluation system similar to the system for therapeutic drugs against Alzheimer's disease, for example, a system using a Y-shaped maze test. Specifically, a drug which induces amnesia, such as scopolamine, alone is administered to a rat or a mouse. Moreover, the composition of the present invention is administered to a rat or a mouse simultaneously with or prior to the administration of such a drug. Then, the mouse or the rat may be subjected to a test using a Y-shaped maze so that an effect of preventing amnesia of the composition of the present invention may be confirmed by using the percentage of change in spontaneous alternation behavior to different arms and the total number of entries into the maze as indicators (see Example 3).

The composition of the present invention includes, as an active ingredient, the peptide Tyr-Pro, and oral administration or oral ingestion thereof allows achievement of the desired effects described above. The period of administration or ingestion of the composition of the present invention or the peptide Tyr-Pro may be variously adjusted upon consideration of the age of a target of the administration or ingestion, such as a human or non-human animal, and the health conditions and the like of the target. Examples of the non-human animal include non-human higher vertebrate animals, particularly non-human animals, including pet animals, such as dogs and cats, and domestic animals, such as cattle, horses, pigs and sheep; however, the non-human animal is not limited thereto. A single administration of the composition of the present invention is enough to demonstrate its effects; however, a continuous effect may be expected by continuous ingestion, which is once or more a day. The composition of the present invention when used as medicine may be in the form of drugs for oral administration. For example, the form may be a tablet, a pill, a hard capsule, a soft capsule, a microcapsule, a powder, a granule, a liquid or the like. When produced as medicine, the composition of the present invention may be produced in a unit dose required for commonly approved drug administration by, for example, including a pharmaceutically acceptable material, such as a carrier, an excipient, a filler, an antiseptic, a stabilizer, a binder, a pH modifier, a buffer, a thickener, a gelatinizing agent, a preservative and an antioxidant, accordingly as needed.

The composition of the present invention may also be used as a material for food and beverage or a material for animal feed. For example, the composition of the present invention or the peptide Tyr-Pro, which is the active ingredient of the composition of the present invention, may be considered a functional food, such as a food for specified health use, which has an effect such as an effect of promoting gastrointestinal motility, an anti-anxiety effect, an effect of promoting blood flow, an effect of preventing amnesia, an effect of regulating heart rate, an effect of regulating the blood glucose, an effect of regulating gastric juice secretion, an effect of regulating body temperature or an effect of stress relaxation.

The dose of administration or ingestion of the present composition or the peptide Tyr-Pro is generally about 0.045 mg/kg weight to 100 mg/kg weight per administration or ingestion in order to obtain desired effects, in terms of the amount of the peptide Tyr-Pro which is the active ingredient. When promotion of gastrointestinal motility is mainly desired, the dose of ingestion of the composition of the present invention is preferably 0.045 mg/kg weight to 0.12 mg/kg weight in terms of the amount of Tyr-Pro. Meanwhile, when an anti-anxiety effect is mainly desired, the amount of Tyr-Pro is particularly preferably 0.1 mg/kg weight to 100 mg/kg weight. When an effect of preventing amnesia is mainly desired, the amount of Tyr-Pro is preferably 1.5 mg/kg weight to 15 mg/kg weight. The dose per ingestion in a food, which is, for example, a functional food, may also be lowered further than the above-described level, depending on the number of ingestions per day. An appropriate dose of ingestion may be further adjusted upon consideration of various factors as described above.

A food, such as a functional food, containing the composition of the present invention or the peptide Tyr-Pro, which is the active ingredient thereof, may be produced by adding, to various kinds of foods, a hydrolysate of a protein containing Tyr-Pro obtained as described above, a condensate thereof, a fermentation product of a raw material containing a protein containing Tyr-Pro itself, or a powdered or granulated product of such a fermentation product. Moreover, if needed, the nutritional balance, flavors and the like of the food may be improved by addition of an additive either: made of other ingredient used in food such as a saccharide, a protein, a lipid, a vitamin, a mineral, and a flavor, which include various carbohydrates, lipids, vitamins, minerals, sweeteners, flavoring agents, coloring agents, texture enhancers and the like, for example; or made of a mixture thereof. Animal feed containing the composition of the present invention or the peptide Tyr-Pro, which is the active ingredient of the composition, may be prepared similarly to food for human consumption.

For example, the above-described functional food may be in the form of a solid, a gel, or a liquid, and may be in the form of, for example, a fermented dairy product such as a lactic acid bacteria beverage, any one of various processed foods and beverages, a dry powder, a tablet, a capsule, a granule, or the like, and, further, may be any of various beverages, yogurt, a liquid food, jelly, a candy, a retort pouch food, a tablet confectionary, a cookie, a sponge cake, bread, a biscuit, a chocolate, or the like.

When a functional food, such as a food for specified health use, containing the composition of the present invention is manufactured, although depending on how the composition has been added and how the food containing the composition is served as a product, the functional food is prepared so that the amount of the peptide Tyr-Pro, which is the active ingredient, to be contained in the final product may be 0.00001 % by weight to 10 % by weight, preferably 0.00003 % by weight to 3 % by weight, further preferably 0.0001 % by weight to 1 % by weight.

Hereinafter, the present invention will be specifically described by way of Examples; however, the scope of the invention is not limited to Examples.

**Example 1**

Effect of Tyr-Pro on Autonomic Nervous Activity

Male Wister rats (approximately 0-week old) (n=3) with a body weight of approximately 300 g were used, and they took food and water ad lib. To examine the activity of the autonomic nerve, after a 3-hour fast, a polyethylene tube for oral administration was inserted into the oral cavity of each rat under urethane anesthesia in the middle of a light period. Thereafter, a sympathetic nerve controlling the adrenal gland, a parasympathetic (vagus) nerve controlling the stomach, or sympathetic nerve in a cutaneous artery of the tail was hooked to a silver electrode, and the electrical activity of the nerve was determined. From the start of the operation to the completion of the determination, the tube was kept inserted in the trachea to secure the airway, and the body temperature (rectal temperature of the rat) was kept at $35.0 \pm 0.5$ °C by using an incubator. In a period where the activities of these nerves were stabilized, 1 ml of each sample was orally administered through a polyethylene tube, and the changes in activities of these nerves were measured. The samples were 0.045 mg/kg weight, 0.12 mg/kg weight, and 1.2 mg/kg weight of Tyr-Pro, as well as 4.2 mg/kg weight of a casein hydrolysate. The casein hydrolysate used was obtained through hydrolysis of a cow's milk casein with an enzyme, and contained 0.5 % by weight or more of Tyr-Pro with respect to the total amount of the casein hydrolysate.

For control 1 ml of the solvent water was orally administered. Data on each of the activities of the sympathetic nerve in the adrenal gland, the parasympathetic nerve in the stomach, and the sympathetic nerve in the cutaneous artery were analyzed in terms of mean of the priming rate per five seconds (pulse/5 s) determined every five minutes, were represented as percentage where the value before the start of the stimulation (value at 0 minutes) was taken as 100%, and expressed in the form of mean $\pm$ standard error. Values for 5 to 60 minutes after the administration were treated as a group, and a significance test with respect to the control group was conducted by the analysis of variance (ANOVA). Figs. 1 to 3 show the results.

When Tyr-Pro was administered at 0.045 mg/kg weight or 0.12 mg/kg weight, the parasympathetic nervous activity in the stomach after the administration was significantly increased in comparison with that of the water-administered group (Fig. 1). In addition, when Tyr-Pro was administered at 0.012 mg/kg weight, the sympathetic nervous activity in the adrenal gland was significantly decreased in comparison with that of the water-administered group (Fig. 2). Furthermore, when Tyr-Pro was administered at 0.12 mg/kg weight, a tendency was observed that the sympathetic nervous activity in the cutaneous artery was lowered in comparison with that of the water-administered group (Fig. 3A). Moreover, when the casein hydrolysate was administered at 4.2 mg/kg weight, the sympathetic nervous activity in the cutaneous artery was significantly decreased in comparison with that of the water-administered group (Fig. 3B). These results revealed that, when orally administered in the range of 0.045 mg/kg weight to 0.12 mg/kg weight, Tyr-Pro promoted the parasympathetic nervous activity in the stomach, suppressed the sympathetic nervous activity in the adrenal gland, and suppressed the sympathetic nervous activity in the cutaneous artery. Moreover, it was revealed that the casein hydrolysate used suppressed the sympathetic nervous activity in the cutaneous artery at 4.2 mg/kg weight.

## Example 2

Effect of Tyr-Pro on Gastric Emptying Function

Male Sprague-Dawley (SD) rats (6-week old) (n=10) were used, and they took food and water ad lib. After preliminary feeding for one week or longer, the rats were subjected to the experiment. Prior to the test, all the rats were fasted in a fasting cage for 24 hours. During the period, the rats took water ad lib. In the test, a solvent (distilled water for injection) and 0.1 mg/kg weight of Tyr-Pro were orally administered to the rats, and, 30 minutes later, a 0.05% dye (phenol red) solution (containing 1.5% of carboxylmethyl cellulose) was orally administered at a volume of 1.5 ml/rat. Each stomach was extirpated under ether anesthesia 20 minutes after the administration of the dye without delay. The extirpated stomach was immersed in a beaker filled with 0.1 N NaOH, finely cut with scissors, and then homogenized by using Polytron. The homogenate was allowed to stand still at room temperature for one hour, and then proteins therein were precipitated by adding a 30% (w/v) trichloroacetic acid solution into the supernatant. The supernatant was centrifuged, and then measured for absorbance at 560 nm. The gastric emptying function (GE: gastric emptying) was calculated in accordance with the following formula.

$$GE\ (\%) = (1 - \text{absorbance of solution recovered from stomachs of}$$

$$\text{test-substance-administered group/absorbance at gastric emptying of 0\%}) \times 100$$

The results are shown in Fig. 4. The measurement results are expressed in the form of mean $\pm$ standard error, and the unpaired Student's t test was used to compare the administered group with the control group. In the gastric emptying function test on the rats, the dye discharge percentage of the control group (distilled water-administered group) was 78.8 $\pm$ 2.41%, whereas the dye discharge percentage of the Tyr-Pro-administered group was 85.4 $\pm$ 2.01%. These results revealed that, when administered at 0.1 mg/kg weight, Tyr-Pro exhibited an effect of promoting the gastrointestinal motility.

## Example 3

Effects of Preventing Amnesia of Tyr-Pro and Casein Hydrolysate

Male ddY mice (approximately 7-week old) (n=15) were used, and they took food and water ad lib. Test substances used were 1.5 mg/kg weight or 15 mg/kg weight of Tyr-Pro, or 200 mg/kg weight or 2000 mg/kg weight of a casein hydrolysate. The casein hydrolysate used was the same as described in Example 1.

The test substances were administered to the mice once orally 60 minutes before the execution of a Y-shaped maze test for evaluation of spontaneous alternation behavior. Further, 30 minutes before the execution of the Y-shaped maze test, 1 mg/kg weight of scopolamine was subcutaneously administered on the backs of the mice in order to induce dysmnesia and/or cognitive impairment in the mice. In the Y-shaped maze test, a Y-shaped maze was used as an experimental device, in which the length of each arm was 40 cm, the height of the wall was 12 cm, the width of the floor was 3 cm, and the width of the upper part was 10 cm and three arms were connected to each other at an angle of 120 degrees. Each of the mice was placed at the tip of any one of the arms of the Y-shaped maze, and then let go to freely explore in the maze for 8 minutes. The sequence of the arms info which each of the mice entered was recorded. The number of entries into the arms of each mouse during the measurement time was counted to be the total number of entries. In the sequence, the combination in which three different arms were selected in succession (for example, with the three arms respectively called A, B, and C, if the sequence of the arms entered is ABCBACACB, the count is 4 inclusive of overlapping) was investigated and the number of the count was used as the number of spontaneous alternation behavior. The percentage of change in spontaneous alternation behavior was calculated by dividing the number of spontaneous alternation behavior by a number obtained by subtracting 2 from the total number of entries and multiplying a resultant number by 100. The percentage of change in spontaneous alternation behavior was used as an indicator of spontaneous alternation behavior. The measured values were expressed in the form of mean $\pm$ standard error for each group. A significant test with respect to the control group was conducted by a Dunnett's multiple comparison test after an one-way analysis of variance. Fig. 5-1 and Fig. 5-2 show the results.

These results revealed that Tyr-Pro exhibited a an effect of preventing amnesia, when administered in a range from 1.5 mg/kg weight to 15 mg/kg weight, and that the casein hydrolysate used exhibited an effect of preventing amnesia, when administered in a range from 200 mg/kg weight to 2000 mg/kg weight.

**Example 4**

Evaluation of Anti-Anxiety Effect of Tyr-Pro by Elevated Plus-Maze Test

Male ddY mice (approximately 6-week old) (n=12) were used. After preliminary feeding, Tyr-Pro (0.1 mg/kg weight, 1.0 mg/kg weight, 10 mg/kg weight, 30 mg/kg weight, or 100 mg/kg weight) or a casein hydrolysate (10 mg/kg weight, 100 mg/kg weight, or 1000 mg/kg weight) was administered once orally. The anti-anxiety effect was examined by an elevated plus-maze test 30 minutes after the administration. The casein hydrolysate was the same as that in Example 1. Diazepam (1.0 mg/kg weight), which is known as an anti-anxiety agent, was administered for a positive control. The elevated plus-maze had such a shape that two vinyl chloride plates each having a length of 65 cm, and a width of 5 cm were crossed over each other into a plus shape, where walls having a height of 50 cm (black acrylic resin) were attached around one of the paths, except the crossing portion at the center. Specifically, the plus-maze included two wall-less arms (open arms), two walled arms (closed arms) and the crossing portion at the center. In the elevated plus-maze test, each of the mice was placed in the crossing portion at the center of the maze in such a manner that the mouse faced one of the walled arms, and then was let go to freely explore in the maze for 5 minutes. The behaviors thereof were observed, and the accumulative time of stay on the open arms was measured. From the measured value, the percentage of the accumulative time of stay on the open arms in a 5-minute test was found, and used as an index of the anti-anxiety effect. Distilled water was administered to a control group and diazepam was administered once orally at 1 mg/kg weight to a positive control group. The measured values were expressed in the form of mean $\pm$ standard error for each group and a comparison with the control group was conducted by using an unpaired Student's t test. The results are shown in Fig. 6.

These results revealed that Tyr-Pro exhibited an anti-anxiety effect in a range of 0.1 mg/kg weight to 100 mg/kg weight and the casein hydrolysate used exhibited an anti-anxiety effect in a range of 10 mg/kg weight to 1000 mg/kg weight.

**References**

Document 1: WO 2002/076455
Document 2: Japanese Patent Application Publication No. 05-247089
Document 3: Izv. Akad. Nauk. Ser. Biol., (2008), No. 1, 61-67
Document 4: WO 2007/030035
Document 5: Japanese Patent No. 3364579

**Claims**

1. A composition for regulating an autonomic nervous activity, comprising, as an active ingredient, Tyr-Pro or a salt thereof.

2. A composition for promoting a parasympathetic nervous activity, comprising, as an active ingredient, Tyr-Pro or a salt thereof.

3. A composition for suppressing an increase in a sympathetic nervous activity, comprising, as an active ingredient, Tyr-Pro or a salt thereof.

4. The composition according to claim 2 or 3, wherein the composition has an effect of promoting gastrointestinal motility, an effect of preventing amnesia, or an anti-anxiety effect.

5. A composition for promoting gastrointestinal motility, comprising, as an active ingredient, Tyr-Pro or a salt thereof.

6. A composition for preventing amnesia comprising, as an active ingredient, Tyr-Pro or a salt thereof.

7. A composition for reducing anxiety, comprising, as an active ingredient, Tyr-Pro or a salt thereof.

8. The composition according to any one of claims 1 to 7, wherein the composition is for oral ingestion.

9. A method for regulating an autonomic nervous activity in a non-human animal, comprising administering to a non-human animal Tyr-Pro or a salt thereof.

10. A method for promoting a parasympathetic nervous activity in a non-human animal, comprising administering to a non-human animal Tyr-Pro or a salt thereof.

11. A method for suppressing an increase in a sympathetic nervous activity in a non-human animal, comprising administering to a non-human animal Tyr-Pro or a salt thereof.

12. The method according to claim 10 or claim 11, wherein
the method is for promoting gastrointestinal motility, preventing amnesia, or reducing anxiety, in a non-human animal.

13. A method for promoting gastrointestinal motility, comprising administering to a non-human animal Tyr-Pro or a salt thereof.

14. A method for preventing amnesia, comprising administering to a non-human animal Tyr-Pro or a salt thereof.

15. A method for reducing anxiety, comprising administering to a non-human animal Tyr-Pro or a salt thereof.

16. The method according to any one of claims 9 to 15, wherein
the administering is oral administration.

## FIG. 1

## FIG. 2

# FIG. 3

(A)

(B)

# FIG. 4

# P<0.1; vs WATER (Student's t – Test )

## FIG. 5-1

(A)

☐ CONTROL GROUP      ▤ YP (1.5mg / kg) GROUP
▨ SCOPOLAMINE CONTROL GROUP      ▩ YP (15mg / kg) GROUP

*** $P < 0.001$ ; vs CONTROL GROUP (Dunnett's multiple comparison test)
# $P < 0.05$, ### $P < 0.001$ ; vs SCOPOLAMINE CONTROL GROUP
(Dunnett's multiple comparison test)

(B)

☐ CONTROL GROUP      ▤ YP (1.5mg / kg) GROUP
▨ SCOPOLAMINE CONTROL GROUP      ▩ YP (15mg / kg) GROUP

* $P < 0.05$ ; vs CONTROL GROUP (Dunnett's multiple comparison test)
## $P < 0.01$; vs SCOPOLAMINE CONTROL GROUP (Dunnett's multiple comparison test)

# FIG. 5-2

## (A)

☐ CONTROL GROUP    ▨ SCOPOLAMINE CONTROL GROUP

☰ CASEIN HYDROLYSATE (200mg / kg) GROUP    ▨ CASEIN HYDROLYSATE (2000mg / kg) GROUP

*** P<0.001 ; vs CONTROL GROUP (Dunnett's multiple comparison test)

\# P<0.05, \#\# P<0.01 ; vs SCOPOLAMINE CONTROL GROUP

(Dunnett's multiple comparison test)

## (B)

☐ CONTROL GROUP    ▨ SCOPOLAMINE CONTROL GROUP

☰ CASEIN HYDROLYSATE (200mg / kg) GROUP    ▨ CASEIN HYDROLYSATE (2000mg / kg) GROUP

*** P<0.001 ; vs CONTROL GROUP (Dunnett's multiple comparison test)

## FIG. 6

\* P<0.05, \*\* P<0.01, \*\*\* P<0.001; vs CONTROL GROUP (Student's t - Test )

**INTERNATIONAL SEARCH REPORT**

| | |
|---|---|
| International application No. | |
| | PCT/JP2010/055413 |

A. CLASSIFICATION OF SUBJECT MATTER

*A61K38/00*(2006.01)i, *A61P1/14*(2006.01)i, *A61P25/02*(2006.01)i, *A61P25/22* (2006.01)i, *A61P25/28*(2006.01)i

According to International Patent Classification (IPC) or to both national classification and IPC

B. FIELDS SEARCHED

Minimum documentation searched (classification system followed by classification symbols)
A61K38/00, A61P1/14, A61P25/02, A61P25/22, A61P25/28

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/MEDLINE/BIOSIS/EMBASE/REGISTRY(STN), JSTPlus/JMEDPlus/JST7580 (JDream2)

C. DOCUMENTS CONSIDERED TO BE RELEVANT

| Category* | Citation of document, with indication, where appropriate, of the relevant passages | Relevant to claim No. |
|---|---|---|
| X | JP 10-095736 A (Calpis Co., Ltd.), 14 April 1998 (14.04.1998), claim 1; paragraph [0001] (Family: none) | 1-8 |
| X<br>Y | WO 2006/084560 A1 (UNILEVER N.V.), 17 August 2006 (17.08.2006), claim 1; table 1<br>& US 2009/0123605 A1 & EP 1879465 A<br>& EP 1845797 A & WO 2006/084573 A1 | 1-8<br>1-4,6-8 |
| Y | ROZZINI, L. et al, Angiotensin converting enzyme (ACE) inhibitors modulate the rate of progression of amnestic mild cognitive impairment, Int J Geriatr Psychiatry, 2006, Vol.21, No.6, p.550-5 | 1-4,6,8 |

☒ Further documents are listed in the continuation of Box C.  ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search<br>09 April, 2010 (09.04.10) | Date of mailing of the international search report<br>20 April, 2010 (20.04.10) |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No.

PCT/JP2010/055413

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 02-022235 A  (E.R. Squibb & Sons, Inc.), 25 January 1990 (25.01.1990), claim 1 & US 4931430 A        & EP 321221 A2 & AU 2640488 A        & CN 1034312 A | 1-4,7,8 |

Form PCT/ISA/210 (continuation of second sheet) (July 2009)

## INTERNATIONAL SEARCH REPORT

| International application No. |
|---|
| PCT/JP2010/055413 |

**Box No. II**      **Observations where certain claims were found unsearchable (Continuation of item 2 of first sheet)**

This international search report has not been established in respect of certain claims under Article 17(2)(a) for the following reasons:

1. ☒ Claims Nos.: 9-16
   because they relate to subject matter not required to be searched by this Authority, namely:
   Claims 9 to 16 pertain to methods for treatment of the human body or animal body by surgery or therapy and thus relate to a subject matter on which this International Searching Authority is not required to carry out a search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**      **Observations where unity of invention is lacking (Continuation of item 3 of first sheet)**

This International Searching Authority found multiple inventions in this international application, as follows:
The inventions in claims 1 - 8 do not have any special technical feature, since the inventions are described in the following documents 1, 2 and cannot be considered to be novel.

    Document 1: JP 10-095736 A (Calpis Co., Ltd.), 14 April 1998 (14.04.1998)
    Document 2: WO 2006/084560 A1 (UNILEVER N.V.), 17 August 2006 (17.08.2006)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**
     ☐ The additional search fees were accompanied by the applicant's protest and, where applicable, the payment of a protest fee.
     ☐ The additional search fees were accompanied by the applicant's protest but the applicable protest fee was not paid within the time limit specified in the invitation.
     ☐ No protest accompanied the payment of additional search fees.

Form PCT/ISA/210 (continuation of first sheet (2)) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2002076455 A
- JP 5247089 A
- WO 2007030035 A
- JP 3364579 B

**Non-patent literature cited in the description**

- *Izv. Akad. Nauk. Ser. Biol.,* 2008, vol. 1, 61-67